Europäisches Patentamt

European Patent Office

Office européen des brevets

(19)

(11) Numéro de publication: **0 238 589**
**B1**

(12)

# FASCICULE DE BREVET EUROPÉEN

(45) Date de publication du fascicule du brevet:
07.02.90

(51) Int. Cl. ⁵: **A 61 B 17/22**, A 61 B 8/08

(21) Numéro de dépôt: 86905851.1

(22) Date de dépôt: 29.09.86

(86) Numéro de dépôt international:
PCT/FR 86/00335

(87) Numéro de publication international:
WO 87/01927 (09.04.87 Gazette 87/08)

(54) **PROCEDE ET DISPOSITIF DE REPERAGE PERMETTANT, AU COURS D'UNE LITHOTRIPSIE, D'APPRECIER LE DEGRE DE FRAGMENTATION DES CALCULS.**

(30) Priorité: 27.09.85 FR 8514330

(43) Date de publication de la demande:
30.09.87 Bulletin 87/40

(45) Mention de la délivrance du brevet:
07.02.90 Bulletin 90/06

(84) Etats contractants désignés:
BE CH DE GB IT LI NL SE

(56) Documents cité:
EP-A-148 653
EP-A-168 559
DE-A-3 119 295
DE-A-3 122 056
DE-A-3 328 066
DE-A-3 348 039

(73) Titulaire: Dory, Jacques
91 rue des Molveaux
F-77450 Coupvray Esblay (FR)

(72) Inventeur: Dory, Jacques
91 rue des Molveaux
F-77450 Coupvray Esblay (FR)

(74) Mandataire: Marquer, Francis
35, Avenue Victor Hugo Résidence Chamfleury
F-78180 Voisins-le-Bretonneux (FR)

EP 0 238 589 B1

LIBERGRAF, STOCKHOLM 1990

## Description

Lors de l'utilisation d'un lithotripteur extracorporel pour la destruction de calculs par ondes de pression, le degré de fragmentation de ceux-ci, dont la connaissance est importante pour déterminer le moment où les tirs doivent être arrêtés, est parfois difficile à apprécier.

En effet, dès le début de la fragmentation, de petits fragments peuvent se déposer sous le calcul et faire écran devant le dispositif de repérage, par exemple échographique, que comporte le lithotripteur. L'image qui apparaît alors sur l'écran de ce dispositif est celle de la couche constituée par le fragment qui se confond alors avec le reste du calcul. La résolution du dispositif n'est pas suffisante pour séparer chaque particule élémentaire et l'opérateur, trompé par l'aspect de l'image, risque d'arrêter prématurément le traitement.

Un autre cas d'erreur est celui où, le calcul étant enclavé dans une cavité, les fragments restent sur place. Il en résulte que l'image échographique peut rester inchangée même lorsque la fragmentation est complète.

L'invention a pour objet un procédé de repérage des calculs et de leurs fragments comportant la formation d'une image et son observation en temps réel, caractérisé en ce que lesdits fragments sont soumis à des ondes élastiques pulsées et focalisées émises avec une puissance relativement faible par rapport à celle des tirs, mais notablement plus élevée que celle utilisée pour l'échographie, et à une cadence de l'ordre de quelques hertz, avantageusement 5 à 10 Hz et que les déformations subies par l'image des fragments sont interprétées pour obtenir une information relative à la taille des fragments.

L'invention part de la découverte du fait que les fragments de faible dimension (inférieure par exemple à 3 mm), soumis à une telle onde élastique, subissent un mouvement d'amplitude inversement proportionnelle à leur taille. En partie, grâce à des phénomènes d'interférence, il en résulte une modification sensible et caractéristique de la forme des échos réfléchis par le fragment, donc de l'image observée. L'écho peut disparaître complètement si la taille du fragment est inférieure à une certaine limite, il sera, par contre, stable pour un fragment de taille supérieure à un seuil déterminé.

Suivant un mode d'exécution préféré, le calcul et ses fragments soumis auxdites ondes élastiques sont observés en échographie de type A effectuée sur une ligne qui passe par la tache focale du faisceau d'ondes élastiques et la disparition ou sa modification de l'écho sont observées sur chacun des fragments pour indiquer que la limite inférieure de taille susvisée est atteinte et qu'il n'est donc plus nécessaire de reprendre les tirs.

Suivant un perfectionnement, une zone contenant des fragments dont on veut analyser les tailles de manière plus fine est soumise, pendant l'application desdites ondes élastiques, à des impulsions élastiques d'examen et la variation de la fréquence porteuse de ces impulsions d'examen par réflexion sur les fragments est mesurée pour donner une indication de la vitesse de déplacement desdits fragments sous l'impact desdites ondes élastiques.

L'invention a encore pour objet un dispositif pour la mise en ouvre du procédé susvisé. Ce dispositif utilise l'émetteur principal d'impulsions ultrasonores du lithotripteur à puissance réduite et à une cadence d'impulsions appropriée à la mise en mouvement des fragments de calculs, et de préférence effectue l'affichage des échos en échographie A.

D'autres particularités, ainsi que les avantages de l'invention, apparaîtront clairement à la lumière de la description ci-après.

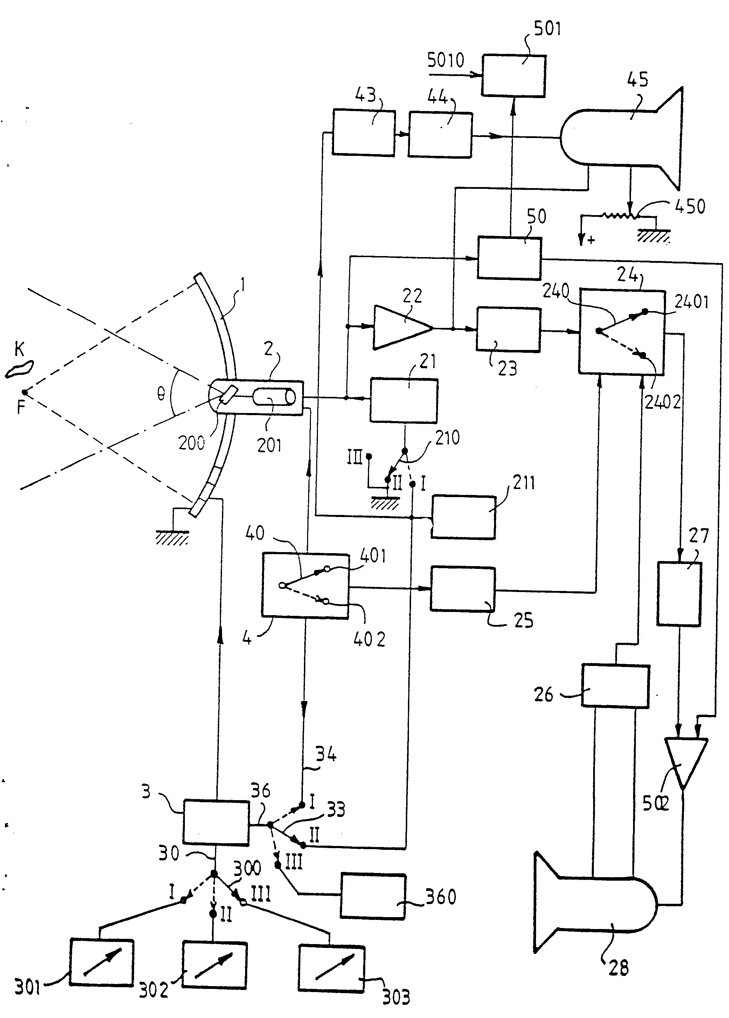

La figure unique du dessin annexé est le schéma de principe d'un lithotripteur équipé d'un appareil de repérage des fragments de calculs conforme à un mode d'exécution préféré de l'invention.

On a représenté au dessin un transducteur 1 en forme de calotte sphérique, réalisé et monté, pour permettre de le positionner suivant trois axes orthogonaux, comme cela a été décrit dans EP-A-0 148 653, déposé au nom de Jacques DORY, pour: "Appareil à impulsions ultrasonores destiné à la destruction des calculs.". Un transducteur auxiliaire 2 est fixé au centre de la calotte sphérique et comporte un élément piézo-électrique oscillant 200 commandé par un moteur 201, lui-même commandé par un circuit électronique 4.

Le transducteur 1 est excité par un émetteur d'impulsions 3 dont une entrée 30 de réglage de la puissance de crête émise est reliée, par l'intermédiaire d'un commutateur 300, soit, en position I, à un organe 301 qui permet le réglage de ladite puissance à une valeur propre à la lithotripsie (de l'ordre de 100 Kw par exemple), soit, en position III, à un organe 303 qui fixe ladite puissance à une valeur beaucoup plus faible propre au repérage de fragmentation (de l'ordre de 10 à 20 Kw par exemple), soit enfin, en position II, à un organe 302 qui fixe ladite puissance à une valeur encore beaucoup plus réduite, de l'ordre de quelques watts. La réalisation des organes symbolisés par les rectangles 301 à 303 est à la portée de l'homme du métier. Pour obtenir la puissance de quelques watts, le bloc 302 devra provoquer une réduction importante de la tension d'alimentation de l'émetteur.

Les ondes engendrées par l'émetteur 3 ont par exemple une haute fréquence de 500 KHz et sont émises, suivant que le commutateur 33 occupe les positions I, II, ou III, soit en impulsions de quelques μs de durée synchronisée par le circuit 4 comme on l'expliquera ci-après, soit sous la forme de 256 impulsions réparties sur une durée de 1/10 s qui correspond à une oscillation complète de l'élément 200, soit enfin sous la forme d'impulsions brèves ayant une cadence de 5 à 10 Hz.

Le circuit 4 engendre des signaux de tension en dents de scie comportant successivement des portions de 1/10 de seconde à pente croissante et des portions de 1/10 de seconde à pente décroissante. Ces portions, qui commandent la rotation du moteur 201 dans un sens puis dans l'autre pour réaliser un balayage sectoriel d'angle θ, sont séparées les unes des autres par des intervalles de 1/100 de seconde pendant lesquels une commande est envoyée sur la sortie 34. Donc, lorsque le commutateur 33 est en position I, lesdites commandes sont reçues sur l'entrée de synchronisation 36 de l'émetteur 3, ce qui déclenche un tir. Par contre, lorsque le commutateur 33 est en position II, l'entrée 36 est reliée à un générateur 211 qui fournit 256 impulsions pendant lesdites portions de 1/10 s. Enfin, lorsque le commutateur 33 est en position III, l'entrée 36 est reliée à un organe 360 qui synchronise l'émetteur à une fréquence d'impulsions de 5 à 10 Hz.

Par ailleurs, la génération de dents de scie par le circuit 4 est commandée par un commutateur 40: les dents de scie sont engendrées lorsque celui-ci est en position 401, interrompues lorsqu'il est en position 402. Le balayage du transducteur 2 est alors arrêté en position médiane du faisceau. Simultanément, la mémoire 24 qui reçoit les échos du transducteur 2 traversés par un amplificateur 22, à travers un convertisseur analogique-numérique 23, est alors bloquée en position "lecture seule", au moyen d'un commutateur 240 qui est couplé (de manière non figurée) au commutateur 40 qui se trouve ainsi alors en position 2402.

Lorsque le commutateur 40 est, par contre, en position 401, le commutateur 240 est en position 2401 et la mémoire 24 fonctionne en écriture et en lecture: les signaux reçus en 22, sont stockés ligne par ligne dans la mémoire 24, un dispositif d'adressage d'écriture 25, commandé par le circuit 4, permettant de faire correspondre les angles respectifs de déviation du faisceau émis et/ou reçu par le transducteur 2 aux lignes respectives de la mémoire. Un dispositif 26 de lecture rapide de la mémoire excite les bobines de déviation en X et en Y d'un tube cathodique 28, donc l'électrode de commande de brillance reçoit le contenu correspondant de la mémoire 24, transformé en signal analogique par un convertisseur numérique-analogique 27.

Le transducteur 2 est relié à un émetteur d'impulsions à très haute fréquence (5 MHz, par exemple) 21 synchronisé, soit par le générateur d'impulsions 211 lorsque le commutateur 210 est en position I, soit mis à la masse lorsque le commutateur 210 est en position II ou III (ces positions I à III étant obtenues en même temps que les positions correspondantes des commutateurs 33 et 300). Dans les positions II ou III des commutateurs, l'émetteur 21 n'est donc pas en service et le transducteur 2 ne fonctionne qu'en réception.

Le signal issu de l'amplificateur 22 est appliqué à la commande de déviation verticale du faisceau d'un deuxième tube cathodique 45, dont la commande de déviation horizontale est assurée par un générateur 44. Ce dernier est lui-même synchronisé par le générateur 211, avec un retard réglable fourni par un circuit de retard 43. Les organes 43 et 44 sont réglés de façon que soit affichée, sur l'écran du tube cathodique 45, une petite portion seulement de la zone du corps contenant le calcul K.

A titre d'exemple, si le calcul se trouve à 100 mm de profondeur, le tube n'affichera qu'une zone correspondant à 20 mm de profondeur, et le retard sera réglé pour correspondre à un parcours de 90 mm par les ondes élastiques. La zone effectivement affichée sera ainsi limitée par des profondeurs comprises entre 90 et 110 mm de profondeur.

Le fonctionnement du dispositif qui vient d'être décrit est le suivant:

Lorsque les commutateurs 33, 300 et 210 sont en position I, des impulsions de grande puissance sont engendrées par le transducteur 1 et focalisées au centre F de la sphère.

Pendant la durée de chaque portion de 1/10 sec., le dispositif d'échographie à balayage sectoriel constitué par le transducteur, l'émetteur auxiliaire et les organes de réception, de traitement et de visualisation 22 à 27, affiche sur l'écran du tube cathodique 28 une image de la zone balayée, donc du rein et du calcul K.

Par ailleurs, le dispositif de visualisation est agencé, de manière connue en soi, pour matérialiser sur l'écran du tube cathodique (par exemple par une croix) la position théorique de la tache focale dans le plan de coupe représenté, plan qui passe par l'axe de symétrie du transducteur 1. (Il s'agit d'échographie du type B). L'opérateur commence par déplacer le transducteur 1 en X, jusqu'à ce que le calcul apparaisse nettement sur l'écran, puis il le déplace en Y et Z, jusqu'à ce que la croix coïncide avec la région centrale de l'image du calcul. A ce moment, les commutateurs peuvent être mis en position II ; la région de la tache focale est alors rendue visible sur l'écran, avec une luminosité proportionnelle à la concentration d'énergie correspondante. On a ainsi une représentation de ce que sera la répartition de l'énergie de l'onde de choc pendant le tir, ce qui permet de contrôler et de parfaire les réglages.

Lorsque l'on veut contrôler la fragmentation, on fait passer les commutateurs de la position I à la position III et le commutateur 40 en position 402. Il en résulte que le balayage est arrêté sur la ligne médiane et que, le commutateur 240 étant lui-même en position 2402, le tube cathodique 28 continue à afficher le calcul. Par ailleurs, le transducteur 1 travaille alors à puissance réduite et à une cadence de 5 à 10 Hz, ce qui a pour effet de provoquer une agitation des fragments de calculs.

Les échos résultant de la réflexion des impulsions émises par le transducteur 1 sont reçus par le transducteur 2 et transmis à la commande de déflexion horizontale du tube cathodique 45, dont l'électrode de commande de luminosité est soumise à une tension continue ajustable au moyen d'un potentiomètre 450. On obtient ainsi une échographie A du calcul, à une échelle telle que l'image des fragments sera bien visible. Cette image est fournie aussi bien lorsque les commutateurs sont en position I que lorsqu'ils sont en position III et, par conséquent, le passage de la position I à la position III permet, pour chaque fragment, d'observer soit l'absence de déforma-tion, soit la déformation ou même la disparition de l'image.

On peut ainsi, en cours de lithotripsie, constater la réduction de taille progressive des fragments en effectuant des commutations successives de I en III.

On a représenté en 50 un dispositif Doppler classique qui émet des impulsions à très haute fréquence (5 MHz par exemple) et qui reçoit les échos correspondants couplés par le transducteur 2. Lorsqu'on veut mettre ce dispositif en service, on place les commutateurs 33, 210 et 300 en position III et le balayage est arrêté en position médiane du faisceau émis par le transducteur 1, le commutateur 40 étant, à cet effet, dans la position 402.

En position III, le transducteur 1 émet des impulsions récurrentes à la fréquence de 500 KHz avec une puissance réduite par rapport à celle des tirs. La cadence de ces impulsions est définie par le générateur 360.

On s'arrange de préférence pour que cette cadence soit un sous multiple de la cadence des impulsions Doppler: cette dernière étant par exemple de 5000 Hz, celle des impulsions de puissance réduite d'agitation des fragments pourra être de 5 - 10 - 20 ou 50 Hz. Le calage relatif des deux trains d'impulsions pourra alors être tel qu'une impulsion Doppler arrive sur le fragment en même temps qu'une impulsion d'agitation.

Les échos Doppler ne sont reçus que pendant un intervalle de temps défini par un créneau engendré à l'intérieur du dispositif 50 de façon connue en soi. Ce créneau est appliqué, ainsi que le signal lu dans la mémoire 24 (alors que le commutateur 240 est en position 2402), à un mélangeur 502 dont la sortie attaque l'électrode de commande de luminosité du tube cathodique 28. On matérialise ainsi, sur l'écran dudit tube, la position d'une fenêtre réglable de sélection d'un ou plusieurs fragments.

Le signal Doppler à basse fréquence engendré par le dispositif 50, et dont l'amplitude est proportionnelle à la vitesse du mouvement d'agitation du fragment, est appliqué à un tube cathodique ou un enregistreur 501, de préférence en même temps que les impulsions d'agitation (appliquées à l'entrée 5010). On peut ainsi observer les corrélations entre ces deux signaux. La fréquence du signal Doppler est inversement proportionnelle à la taille des fragments, ce qui permet une observation quantitative de celle-ci au besoin par analyse spectrale du signal Doppler.

La mesure Doppler et le repérage des fragments par échographie A peuvent être effectués simultanément.

Il va de soi que diverses modifications de détail pourront être apportées aux dispositifs décrits et représentés. On peut même envisager de supprimer l'un des deux moyens de repérage de la taille des fragments, ou encore, de remplacer l'échographie A par un autre moyen d'imagerie en temps réel, par exemple, un dispositif à rayons X associé à un amplificateur de brillance.

**Revendications**

1. Procédé de repérage des fragments de calculs obtenus au cours d'une lithotripsie par ondes de pression, comportant la formation d'une image et son observation en temps réel, caractérisé en ce que lesdits fragments sont soumis à des ondes élastiques pulsées et focalisées émises avec une puissance relativement faible par rapport à celle des tirs, mais notablement plus élevée que celle utilisée pour une échographie, et à une cadence de quelques hertz et que les déformations subies par l'image des fragments sont interprétées pour obtenir une information relative à la taille de ceux-ci.

2. Procédé selon la revendication 1, caractérisé en ce que le calcul et ses fragments, soumis auxdites ondes élastiques, sont observés en échographie de type A effectuée sur une ligne qui passe par la tache focale du faisceau d'ondes élastiques et la disparition de l'écho est observée sur chacun des fragments pour indiquer que la limite inférieure de taille susvisée est atteinte et qu'il n'est donc plus nécessaire de reprendre les tirs.

3. Procédé selon la revendication 1 ou 2, caractérisé en ce qu'une zone contenant des fragments dont on veut analyser les tailles de manière plus fine est soumise, pendant l'application desdites ondes élastiques, à des impulsions élastiques d'examen et la variation de la fréquence porteuse de ces impulsions d'examen par réflexion sur les fragments est mesurée pour donner une indication de la vitesse de déplacement desdits fragments sous l'impact desdites ondes élastiques.

4. Appareil pour la mise en oeuvre du procédé selon la revendication 1 associé à un lithotripteur comprenant un générateur d'impulsions ultrasonores de puissance comportant un transducteur piézo-électrique principal (1) dont la surface active est une coupelle sphérique, ledit appareil comportant des moyens (33 - 360 - 300 - 303) de commuter les réglages de puissance et de cadence de l'émetteur d'impulsions (3) associé au transducteur principal (1) successivement sur des valeurs appropriées à la génération desdites impulsions ultrasonores de puissance et desdites ondes élastiques pulsées et focalisées.

5. Appareil pour la mise en oeuvre du procédé selon la revendication 2, et conforme à la revendication 4, dans lequel le lithotripteur comporte en outre un dispositif d'échographie comprenant un générateur auxiliaire (21 - 211) d'impulsions ultrasonores d'échographie associé à un transducteur auxiliaire (2) solidarisé à ladite coupelle sphérique (1), et effectuant un balayage sectoriel, ledit appareil comportant des moyens (210) de mettre hors service l'émetteur (21) dudit générateur d'impulsions d'échographie, des moyens (40) d'interrompre le balayage du transducteur auxiliaire en position médiane et des moyens (45) d'afficher en échographie A les échos reçus par ledit transducteur auxiliaire lorsque le transducteur principal (1) engendre lesdites ondes élastiques pulsées et focalisées.

6. Appareil selon la revendication 5, caractérisé en ce que lesdits moyens d'affichage en échographie A comportent en outre des moyens (43 - 44) de régler une fenêtre d'affichage desdits échos reçus par le transducteur auxiliaire pour obtenir l'affichage à grande échelle, sur l'écran d'un tube cathodique (45), d'une zone d'observation qui contient le calcul et ses fragments.

7. Appareil pour la mise en oeuvre du procédé selon la revendication 3, caractérisé en ce qu'il est associé à un lithotripteur du type défini aux revendications 4 et 5 et comporte un dispositif émetteur récepteur Doppler (50) mis en service pendant l'émission, par le transducteur principal (1) desdites ondes élastiques pulsées et focalisées et couplé audit transducteur auxiliaire (2), pendant que ledit émetteur (21) est hors service.

8. Appareil selon la revendication 7, caractérisé en ce que ledit dispositif Doppler (50) engendre une fenêtre d'observation des fragments, que le lithotripteur comporte un tube cathodique (28) d'affichage des échos de repérage du calcul formés par ledit dispositif d'échographie et que des moyens (502) sont prévus pour que ladite fenêtre d'observation soit affichée sur l'écran dudit tube cathodique (28) en même temps que lesdits échos de repérage du calcul.


**Patentansprüche**

1. Verfahren zur Erkennung von Steinfragmenten, die während einer Steinzertrümmerung durch Druckwellen erhalten wurden, das die Erzeugung eines Bildes und seine Beobachtung in Realzeit enthält, dadurch gekennzeichnet, dass diese Fragmente elastischen gepulsten und fokalisierten Wellen unterworfen werden, die mit einer Leistung, die relativ schwach in bezug auf die der Beschüsse, aber deutlich grösser als die für eine Ultraschalluntersuchung verwendete Leistung ist, und mit einer Kadenz von einigen Hertz ausgesandt werden, und dass die Verformungen, denen das Bild der Fragmente unterworfen ist, so ausgewertet werden, dass man eine Information in bezug auf die Grösse der Fragmente erhält.

2. Verfahren nach Anspruch 1, dadurch gekennzeichnet, dass der Stein und seine Fragmente, die den obengenannten elastischen Wellen unterworfen werden, in einer Ultraschalluntersuchung vom Typ A beobachtet werden, die auf einer Linie durchgeführt wird, welche durch den Brennpunkt des Bündels elastischer Wellen verläuft, und das Verschwinden des Echos auf jedem der Fragmente beobachtet wird, um anzuzeigen, dass die untere Grössengrenze erreicht und es daher nicht mehr notwendig ist, die Beschüsse zu wiederholen.

3. Verfahren nach Anspruch 1 oder 2, dadurch gekennzeichnet, dass ein Bereich mit Fragmenten, deren Grösse man genauer analysieren möchte, während der Anwendung dieser elastischen Wellen elastischen Untersuchungsimpulsen unterworfen wird, und die Veränderung der Trägerfrequenz dieser Untersuchungsimpulse durch Reflektion auf die Fragmente gemessen wird, um die Bewegungsgeschwindigkeit dieser Fragmente beim Auftreffen dieser elastischen Wellen anzuzeigen.

4. Gerät zur Anwendung des Verfahrens nach Anspruch 1, das einem Steinzertrümmerer zugeordnet ist, der einen Leistungs-Ultraschallimpuls-Generator mit einem piezoelektrischen Haupttransduktor (1) aufweist, dessen aktive Oberfläche eine kugelförmige Schale ist, wobei dieses Gerät Mittel (33 - 360 - 300 - 303) enthält, um die Leistungs- und Taktsteuerungen des dem Haupttransduktor (1) zugeordneten Impulssenders (3) nacheinander auf Werte umzuschalten, die zur Erzeugung dieser Leistungs-Ultraschallimpulse und dieser gepulsten und fokalisierten elastischen Wellen geeignet sind.

5. Gerät zur Anwendung des Verfahrens nach Anspruch 2, und entsprechend Anspruch 4, bei dem der Steinzertrümmerer ausserdem eine Ultraschallvorrichtung mit einem Hilfsgenerator (21 - 211) für Ultraschallimpulse enthält, der einem Hilfstransduktor (2) zugeordnet ist, welcher mit der kugelförmigen Schale (1) verbunden ist und ein sektorielles Überstreichen durchführt, wobei das Gerät Mittel (210) aufweist, um den Sender dieses Ultraschallimpuls-Generators auszuschalten, Mittel (40), um das Überstreichen des Hilfstransduktors in der mittleren Stellung zu unterbrechen und Mittel (45), um im Ultraschall A die vom Hilfstransduktor empfangenen Echos aufzuzeigen, wenn der Haupttransduktor (1) die elastischen, gepulsten und fokalisierten Wellen erzeugt.

6. Gerät nach Anspruch 5, dadurch gekennzeichnet, dass die Ultraschall A Anzeigemittel weiter Mittel (43 - 44) aufweisen, um ein Anzeigefenster für diese vom Hilfstransduktor empfangenen Echos zu steuern, um die Anzeige in grossem Masstab auf dem Bildschirm einer Kathodenröhre (45) eines Beobachtungsbereichs zu erhalten, der den Stein und seine Fragmente enthält.

7. Gerät zur Anwendung des Verfahrens nach Anspruch 3, dadurch gekennzeichnet, dass es einem in den Ansprüchen 4 und 5 definierten Steinzertrümmerer zugeordnet ist und eine Doppler-Sender-Empfängervorrichtung (50) aufweist, die, während der Haupttransduktor (1) besagte elastische gepulste und fokalisierte Wellen sendet, in Betrieb gesetzt wird und mit dem Hilfstransduktor (1) gekoppelt ist, während besagter Sender (21) ausser Betrieb ist.

8. Gerät nach Anspruch 7, dadurch gekennzeichnet, dass besagte Doppler-Vorrichtung (50) ein Fenster zur Beobachtung der Fragmente erzeugt, dass der Steinzertrümmerer eine Kathodenröhre (28) zur Anzeige der vom besagten Ultraschallgerät gebildeten Steinerkennungsechos enthält, und dass Mittel (502) vorgesehen sind, damit das Beobachtungsfenster auf dem Bildschirm dieser Kathodenröhre (28) zur gleichen Zeit wie die Steinerkennungsechos angezeigt wird.

## Claims

1. A method of locating fragments of stones obtained during pressure wave lithotrity, comprising the formation of an image and observation thereof in real time, characterized in that said fragments are subjected to pulsed and focused elastic waves emitted with a relatively small power with respect to that of the firing, but appreciably higher than that used for echography, and at a rate of a few Hz and in that the deformations undergone by the image of the fragments are interpreted so as to obtain information relative to the size thereof.

2. Method according to claim 1, characterized in that the stone and its fragments, subjected to said elastic waves, are observed with type A echography effected on a line which passes through the focal spot of the elastic wave beam and the disappearance of the echo is observed on each of the fragments so as to indicate that the above mentioned lower limit of size has been reached and that it is therefore no longer necessary to resume firing.

3. Method according to claim 1 or 2, characterized in that a zone containing fragments whose sizes it is desired to analyse more finely, is subjected, during the application of said elastic waves, to elastic examination pulses and the variation of the carrier frequency of these examination pulses by reflection from the fragments is measured so as to give an indication of the speed of movement of said fragments under the impact of said elastic waves.

4. Apparatus for implementing the method according to claim 1 associated with a lithotriptor including an ultrasonic pulse power generator having a main piezoelectric transducer (1) whose active surface is a spherical cup, said apparatus including means (33 - 360 - 300 - 303) for switching the power and rate adjustments of the pulse emitter (3) associated with the main transducer (1) successively to values appropriate to the generation of said ultrasonic power pulses and said pulsed and focused elastic waves.

5. Apparatus for implementing the method according to claim 2, and in accordance with claim 4, in which the lithotriptor further includes an echography device having an auxiliary ultrasonic echography pulse generator (21 - 211) associated with an auxiliary transducer (2) coupled to or integral with said spherical cup (1) and providing sectorial scanning, said apparatus including means (210) for placing the emitter (21) of said echography pulse generator out of service, means (40) for interrupting the scanning of the auxiliary transducer in the median position and means (45) for providing an echography A display of the echoes received by said auxiliary transducer when the main transducer (1) generates said elastic pulsed and focused waves.

6. Apparatus according to claim 5, characterized in that said echography A display means further include means (43 - 44) for adjusting a display window for said echoes received by the auxiliary transducer so as to obtain the large scale display, on the screen of a cathode ray tube (45), of an observation zone which contains the stone and its fragments.

7. Apparatus for implementing the method of claim 3, characterized in that it is associated with a lithotriptor of the type defined in claims 4 and 5 and includes a Doppler emitting-receiving device (50) brought into service during emission, by the main transducer (1) of said pulsed and focused elastic waves and coupled to said auxiliary transducer (2) while said emitter (21) is out of service.

8. Apparatus according to claim 7, characterized in that said Doppler device (50) generates a window of observation of the fragments, in that the lithotriptor includes a cathode ray tube (28) for displaying the stone locating echoes formed by said echography device and in that means (502) are provided for said observation window to be displayed on the screen of said cathode ray tube (28) at the same time as said stone locating echoes.